# EUROPEAN PATENT SPECIFICATION

(11) **EP 3 754 550 B1**
(45) Date of publication and mention of the grant of the patent: **14.10.2026**
(21) Application number: 19181607.3
(22) Date of filing: 21.06.2019
(51) Int. Cl.: G06V 10/776

(54) **METHOD FOR PROVIDING AN AGGREGATE ALGORITHM FOR PROCESSING MEDICAL DATA AND METHOD FOR PROCESSING MEDICAL DATA**
VERFAHREN ZUR BEREITSTELLUNG EINES AGGREGATALGORITHMUS ZUR VERARBEITUNG VON MEDIZINISCHEN DATEN UND VERFAHREN ZUR VERARBEITUNG VON MEDIZINISCHEN DATEN
PROCÉDÉ DE FOURNITURE D'UN ALGORITHME D'AGRÉGAT POUR LE TRAITEMENT DE DONNÉES MÉDICALES ET PROCÉDÉ DE TRAITEMENT DE DONNÉES MÉDICALES

(43) Date of publication of application: 23.12.2020
(73) Proprietor: Siemens Healthineers AG, 91301 Forchheim (DE)
(72) Inventor: Schmidt, Christian, 90402 Nürnberg (DE); Schöbinger, Max, 96114 Hirschaid (DE); Wels, Michael, 96047 Bamberg (DE)
(74) Representative: Siemens Healthineers Patent Attorneys

(56) References cited:
- EP-A2- 3 101 599
- US-A1- 2018 018 590

## Description

The invention concerns a method for providing an aggregate algorithm for processing medical image data and a method for processing medical image data. Additionally, the invention concerns a system, a computer program and a computer-readable storage medium.

In recent years there has been an upswing in interest and research in the areas of artificial intelligence and machine learning. Technologies such as deep learning enabled a significant quality leap in fully automated algorithms for segmenting and classifying image data, allowing such algorithms to be used in medical data processing and especially the processing of medical image data. The most common approach used in machine learning is supervised learning that requires training data to train an algorithm. Typically, it is necessary to manually identify at least the features that need to be detected and/or qualified in the training data and optionally also certain features that are evaluated by the algorithm to reduce the complexity of the data and make patterns more visible for the learning process. In deep learning the algorithm itself learns the high-level features from annotated data in an incremental manner. Hence the performance of the algorithm depends to a large extend on the amount of data and the quality of annotations being used to train the algorithm. This is especially true since a multitude of variables need to be taken into account, e.g. an anatomical and/or pathological variability between the patients and/or a variability of the used imaging procedures or other acquisition protocols.

It is therefore a major challenge for the manufacturers of medical devices and other suppliers of such trained algorithms to assemble a sufficiently large and sufficiently well annotated set of training data. It can e.g. be necessary to use more than 10.000 or more than 100.000 sets of training data. In medical imaging there are also major privacy concerns, since medical image data and other medical data concerns highly private information. Therefore, an extensive anonymisation protocol needs to be implemented and it can be an elaborate process to gain access to large datasets that could e.g. be provided by hospitals. Once a sufficiently large dataset is acquired it is also typically necessary to manually annotate this extensive dataset by in-house experts or contractors. This might also require an acquisition of relevant knowledge.

Further the data processing can include the transfer of data across national borders and therefore additional requirements concerning data protection, especially considering a multitude of country-specific regulations. An inclusion of third parties for data processing typically requires the agreement of the original patients. These additional limitations limit the possibilities of outsourcing and data sharing.

The overall result of the issues mentioned above tends to lead to slow update and feedback cycles for products using machine learning.

From the document US 2014/0219548 A1 it is known to use local machine learning on an end user system. While this approach allows for an immediate improvement of the algorithm depending on user feedback, the algorithm can only profit from the local training. Since the pool of locally available training data is typically rather small, only a slow improvement of the algorithm is possible. The local algorithm can also not profit from any training performed in other locations. For processing medical data it is also desirable to perform a validation of the trained algorithm before the algorithm is released and used, to ensure that there is no unexpected behaviour of the algorithm. This is typically performed by the provider of the algorithm, e.g. the manufacturer of the medical device used. There is no simple approach for integrating such a validation in the approach used for local learning discussed in the mentioned document.

A different approach to using local data for training an algorithm is discussed in the document US 2018/0018590 A1. This document suggests to generate proxy data on the local system that has a similar distribution of data as the actual data. This proxy data is then aggregated from multiple systems and used to train the algorithm. While this approach improves the privacy compared to a direct redistribution of the local private data, transmitting information about the distribution of certain private data from e.g. a local hospital might already be problematic, especially if some of the data concerns a rare condition. Therefore, additional steps would be necessary to ensure that no privacy relevant data is accidently shared by the method proposed in this document. The creation of artificial data distributions that are similar to the true data distribution can also be problematic since algorithms used in machine learning, e.g. neural networks, can be highly nonlinear systems. A distribution that looks similar can therefore cause vast differences in the resulting trained algorithm. While the document suggests to check for this fact by using a local training using the real data and the proxy data and comparing the trained algorithms, it can not be guaranteed, that this similarity of behaviour still holds true when multiple proxy datasets are merged and used for training.

It is therefore the object of the present invention to provide a method for providing an aggregate algorithm for processing medical data and therefore also to provide a method for processing medical data that allows for the use of local training data while at the same time ensuring that privacy concerns about this data are respected.

The invention solves this problem by providing a method for providing an aggregate algorithm for processing medical image data according to claim 1, a for processing medical data according to claim 7, a system according to claim 8 and a computer program according to claim 10. According to the method for providing an aggregate algorithm for processing medical image data, a multitude of local algorithms is trained by machine learning, wherein the training of each respective local algorithm is performed on a respective local system using respective local training data, wherein a respective algorithm dataset concerning the respective local algorithm is transferred to an aggregating system that generates the aggregate algorithm based on the algorithm datasets (e.g. a plurality algorithm datasets of respective local algorithms). The method can be computer-implemented on an aggregating system which can comprise appropriate interfaces for receiving input data (e.g. the respective local algorithm) and providing output data (e.g. the aggregate algorithm)

The training of each respective local algorithm itself can be provided as a (computer-implemented) method. It can comprise:
- receiving input training data, e.g. with a first training interface),
- receiving output training data, e.g. with a second training interface), wherein the output training data is related to the input training data,
- training a function based on the input training data and the output training data, e.g. with a training computation unit, and
- providing the trained function, e.g. with a third training interface.

In this approach it is possible to only use the local training data locally to train the respective local algorithm. Therefore, the local training data that typically comprises private information or any information that allows drawing conclusions about such private information does not need to be transferred to the aggregating system. In machine learning approaches it is typically not possible to reconstruct the training data from the trained algorithm. Therefore, the suggested method implicitly implements protection of the private data that might be part of the local training data. The suggested method therefore allows for using local training data while automatically respecting any privacy concerns regarding this data. Preferably none of the local training data is transferred to the aggregating system, e.g. from a local hospital to the manufacturer of a medical device or some other provider of algorithms for the processing of medical data. The aggregate algorithm can be independent of the local training data except for the indirect dependence via the algorithm dataset.

The algorithm dataset can describe the complete local algorithm or parameters that are used to parametrize a given basic algorithm. Such parameters can e.g. be the input weights of individual nodes of a neuronal network.

The output of the local algorithm and/or the aggregate algorithm can e.g. be a classification of a depicted feature, a segmentation of an image or information concerning this segmentation, a probability of a certain medical condition etc. The local algorithm and the aggregate algorithm can provide the same type of output data. It is however also possible that the aggregate algorithm is a more complex algorithm that comprises at least one sub-algorithm generated in dependence of the local algorithms. It might e.g. be possible to only use the local training data to locally train the local algorithm for edge detection and use the respective algorithm datasets describing these local algorithms to generate an aggregate algorithm that performs a more complex segmentation task. It is also possible to use the local machine learning to teach a multitude of local algorithms that are than used to provide different sub-algorithms of the aggregate algorithm.

The local algorithm can be a neural network, especially a deep neuronal network, a decision tree, e.g. a random tree, or any other kind of algorithm usable in machine learning. Multitudes of such algorithms are known in the prior art and will therefore not be discussed in detail. The local algorithm can be completely described by the algorithm dataset or by the algorithm dataset and given prior information that can e.g. describe the given basic algorithm. In the second case the algorithm dataset can describe the parameters used to parametrize the given basic algorithm.

In general, a trained function or algorithm mimics cognitive functions that humans associate with other human minds. In particular, by training based on training data the trained function is able to adapt to new circumstances and to detect and extrapolate patterns.

In general, parameters of a trained function or algorithm can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, a trained function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

The local algorithm and the algorithm dataset should not allow for the recovery of the local training data. This is typically true when the amount of data in the local training dataset is sufficiently large compared to the number of parameters in the algorithm dataset. It is especially true for training neural networks and most types of decision trees.

For a few rare cases, e.g. the k-nearest neighbours algorithm, some part of the training data might be recovered. It is preferable to avoid the use of such algorithms for the local training.

The local system does not necessarily need to consist of a single computer or unit, e.g. a work station or a control unit in a medical imaging device. It is also possible that the local system can comprise one or multiple servers and even use a distributed data storage, even at remote locations. The local system can typically be assigned to a certain location and/or group of users. It can e.g. be the data processing system of a hospital or a group of hospitals.

The aggregating system can e.g. be operated by a manufacturer of medical devices or a provider of algorithms trained by machine learning. It can be a local system, e.g. a work station, a server, a set of multiple servers, implemeted in a cloud, etc.

At least part of the local training data can be generated by applying an initial algorithm to local input data to generate local output data, providing a local data representation that depends on the local output data to a user and storing a user feedback provided by the user or a user information determined from the user feedback as part of the local training data. The local training and therefore as a result the aggregate algorithm can therefore take user feedback to the initial algorithm into account. This is especially useful when the method is used as part of an iterative process, wherein the aggregate algorithm is then rolled out as a new initial algorithm and therefore continuously improved based on the user feedback.

The user feedback can be a simple rating of the quality of the output data or the data representation. E.g. the user can assign a grade or a number of stars, e.g. between one and five stars, to the result. This can be directly used for reinforcement learning. E.g. the local algorithm can be slightly varied and the result of the variation can be rated to implement a reinforcement learning.

It is however also possible to use such ratings to train each local algorithm to recognize and rate good and bad outputs and aggregate these local algorithms, e.g. by using a majority voting or an averaging of their outputs. A reinforcement learning of the aggregate algorithm can then be implemented by varying the aggregate algorithm and rating the respective output of each variation by the aggregated local algorithms to create feedback for the reinforcement learning.

Additionally or alternatively it is possible to acquire changes to the output data or the data representation by the user as user feedback. E.g. a user might modify a segmentation that is output by the initial algorithm. This modified segmentation and the input data can then be used as input and output data during a supervised training of the local algorithm.

The training datasets can comprise additional data, e.g. the input data and/or the output data of the local algorithm and/or further data, e.g. patient data, data concerning the imaging conditions, etc. The input data can be locally acquired. The acquisition can be performed outside of the claimed method or in an additional step of the claimed method. The acquisition can be performed by a medical imaging device, e.g. by a computer tomograph or another device based on x-ray imaging, by a magnetic resonance tomograph or any other type of medical imaging device or sensors used during medical data acquisition. The local system can be the medical imaging device or a device used to acquire the medical data or a device to which that data is provided, e.g. a hospital database.

The aggregate algorithm can be updated in multiple iterations, wherein at least one of the iterations uses the aggregate algorithm determined during a previous iteration as the initial algorithm for the current iteration. This allows for a continuous improvement of the locally used algorithm. The respective initial algorithm used during a certain iteration can be an algorithm used to provide output data during the normal every day operation of e.g. a certain medical device. It can be updated at each iteration, especially when an updated aggregate algorithm is rolled out by the manufacturer of the device or another provider of the algorithm. During the normal operation, the collection of local training data and the training of the local algorithm can be performed and used to feedback the algorithm dataset that is then used to improve the initial algorithm in the next iteration.

The initial algorithm can be trained by machine learning using initial training data. If the aggregate algorithm is updated in multiple iterations as discussed above, it is especially possible that the initial algorithm during the first iteration is exclusively trained by initial training data that does not comprise any of the local training data and wherein the initial training is independent of any algorithm datasets generated during local training. In other words, the initial algorithm used during the first iteration or used before the aggregate algorithm is generated can be generated by a normal approach to machine learning, namely by using a sufficiently large, especially annotated and anonymized, dataset to perform an initial training. This initial training can then be improved by the discussed method of using a combination of local training and an aggregate algorithm.

At least part of the initial training data can be provided to the aggregating system and used to validate the aggregate algorithm and/or to determine a respective quality measure for each of the local algorithms, wherein the quality measures are used to generate the aggregate algorithm. Since the provider of the aggregate algorithm, e.g. a manufacturer of medical devices, has no or only limited control concerning the local training data and therefore the local training, it is advantageous to ensure that the aggregate algorithm does not show any unexpected behaviour and meets certain quality requirements. It can e.g. be tested if the aggregate algorithm performs at least as well as or at least within a certain margin of tolerance of the initial algorithm when applied to the initial training data or a certain selected part of the initial training data.

It is also possible to check the quality of the local algorithms before generating the aggregate algorithm, e.g. by applying the different local algorithms to at least part of the initial training data to determine the quality measure. It is then e.g. possible to only use some of the local algorithms selected depending on the respective quality measures to generate the aggregate algorithm or to e.g. use different weights when combining the different local algorithms into the aggregate algorithm. It is e.g. possible that the aggregate algorithm comprises multiple of the local algorithms, wherein the outputs of the local algorithms are averaged and/or a majority voting for the output is performed. In this case the quality measure can be used to determine a weight for the different local algorithms. E.g. a higher weight can be used when the local algorithm performs well on the initial training data or on a certain part of the initial training data.

At least part of the initial training data can be provided to each of the local systems and used to train the local algorithm. The local algorithm can therefore be trained using the local training data and at least part of the initial training data. This allows using a supervised local training even if the amount of available local training data is relatively small. It is e.g. possible to automatically install all or at least part of the initial training data on the local system when e.g. software for processing the medical data is installed or before a medical device is delivered to the customer. The installed part of the initial training data can then be combined with the local training data to provide a larger set of training data that can be used to train the local algorithm.

Instead of performing a completely new supervised learning for the local algorithm it is also possible to use approaches that can modify an initial algorithm based on new training data. An example of such an approach is e.g. to discuss already cited document US 2014/0219548 A1.

The user feedback can comprise a modification of the local data representation and/or the local output data and/or a rating concerning the quality of the local data representation and/or the local output data. Various uses for this kind of user feedback were already discussed previously.

The aggregate algorithm can comprise the evaluation of at least some of the local algorithms, wherein the outputs of the individual local algorithms are combined by a weighted or non-weighted averaging and/or majority voting. This was already discussed above. It is especially possible that the weighting can be determined based on the previously discussed quality measure.

It is also possible to generate an aggregate algorithm that does not comprise the evaluation of individual local algorithms. It is e.g. possible to use a user feedback that rates the quality of the local output data or the resulting local data representation, use pairs of input data and output data and the respective rating as training data to train an algorithm designed to rate the output data of the aggregate algorithm or a certain sub-algorithm of the aggregate algorithm and then use this algorithm to generate feedback during reinforcement learning of the aggregate algorithm. The discussed approaches to generating or training the aggregate algorithm are just some possible examples for this process. Any other approach that can generate an aggregate algorithm based a multitude of trained algorithms and/or algorithm datasets concerning these algorithms could be used.

The local algorithm and/or the aggregate algorithm and/or the initial algorithm can process image data as input data and output a segmentation of the image data and/or parameters concerning the anatomy of a patient depicted by the image data. Parameters concerning the anatomy can e.g. concern certain organs and/or vessels. It is also possible to detect and/or parametrize anomalies and/or to provide information about possible medical conditions. A possible output can e.g. be a segmented vessel or vessel tree, a tracing of a coronary, a relevant output of a CT-angio-application etc.

The invention also concerns an aggregate algorithm provided by the previously discussed method and a computer-readable storage medium containing information representing this algorithm. Information representing this algorithm can be a program or an instruction set implementing the trained algorithm or it can provide parameters determined during the training that parametrized a given algorithm comprising multiple free parameters, e.g. a neural network.

The described method can further be improved, when an incentive mechanism is used to motivate users of local systems to provide the algorithm dataset to a party providing the aggregate algorithm, e.g. a manufacturer of medical devices. It could e.g. be possible to offer updates of the aggregate algorithm only to users providing algorithm datasets or to provide such updates or updates of a program comprising the aggregate algorithm at a reduced price or for free for such users. Alternatively training courses, financial compensations etc. could be offered.

When user feedback is evaluated as part of the local training data this should typically be performed automatically. It might therefore be required that the feedback or any annotations of input data or output data need to confirm to certain technical requirements, e.g. be provided in a certain format. It is therefore advantageous, when the method comprises the step of providing recommendations about formatting or other technical requirements of the user feedback, e.g. annotations, to user prior to the submission of a current set of local training data.

While the quality of the individual local algorithms and/or the quality of the resulting aggregate algorithm can be validated as discussed above, it might still be advantageous to limit the local training to selected sites, user groups or individual users. If e.g. a segmentation algorithm should be improved by the method only a selected group of experts and their local systems could be used for local training.

The invention also concerns a method for processing medical data, especially medical image data, wherein the medical image data is processed by an aggregate algorithm that is generated by the inventive method discussed above to generate output data. The previously mentioned training is not a necessary part of the method for processing medical data, but the usage of the trained aggregate algorithm is part of this method.

Thus, in the most general terms, this aspect of the invention provides a method for providing output data, namely processed medical image data. It can be a computer-implemented method and it can e.g. comprise the steps of:
- receiving input data, e.g. with a further first interface),
- applying a trained function / function trained by a machine learning algorithm (i.e. the aggregate algorithm) to the input data, e.g. with a computation unit, wherein the output data is generated, and
- providing the output data, e.g. with a further second interface.

It is especially advantageous when the method for processing medical data is simultaneously used to improve the aggregate algorithm by generating local training data or when a method for processing medical data using an initial algorithm is used to provide local training data, wherein the respective local training data can then be used to generate the aggregate algorithm as discussed above.

E.g. a local system can run an application for processing medical data that uses the initial algorithm or a first version of the aggregate algorithm to generate output data. The application can provide a user interface element by which the user can manually submit the current case to the local training data that can then be used to train the local algorithm as discussed above and therefore to generate or improve the aggregate algorithm. Alternatively this submission can be automatically performed for each case.

The user can e.g. rate the generated output data or data representation or provide other feedback, e.g. a modification of the results of the algorithm, e.g. a change to the segmentation. This user feedback can then be used as discussed above to train the local algorithm and therefore to generate the aggregate algorithm. Integrating the method for providing an aggregate algorithm closely with the method for processing medical data therefore allows users to actively participate in the improvement of the algorithms used for processing medical data with no or at least a minimal amount of additional effort. Due to the previously discussed method it can be ensured that it is not necessary to communicate any private data to the aggregating system that can e.g. be operated by the manufacturer of used medical device or some other service provider. The operator of the aggregating system can then e.g. validate the improved aggregate algorithm and then provide it as a free or paid update to the users. An updated algorithm can be rolled out as an update that only changes this algorithm or as part of an update to the application for processing medical data.

The invention also concerns a system comprising at least one processing unit configured to perform the function of the aggregating system in the inventive method for providing an aggregate algorithm and/or to perform the method for processing medical data. If the processing unit is configured to perform a method for processing medical data it can e.g. be integrated in a medical imaging device or be a work station used to evaluate the data from the medical imaging device. It is however also possible to perform this function e.g. by a server, a network comprising multiple servers or a cloud solution. The aggregating system will in many use cases require a relatively large amount of resources and can therefore e.g. be implemented on a server or a network of multiple servers or as a cloud solution. In some applications it can however also be advantageous to implement this functionality on a workstation or within a medical imaging system.

When the system comprises a processing unit configured to perform the function of the aggregating system it can additionally comprise multiple further processing units that are configured to perform the functions of the local systems in the method for providing an aggregate algorithm and/or to perform the method for processing medical data. In this case the system can especially be configured to implement the complete method for providing an aggregate algorithm. It is especially possible that the local systems are also used to process medical data as discussed above.

The invention further relates to a computer program that can be directly loaded into a memory unit of a processing unit, the computer program comprising instructions for performing the function of the aggregating system in the method for providing an aggregate algorithm and/or for performing the method for processing medical data when the program is executed on a processing unit.

The invention further relates to a computer-readable storage medium containing electronically readable instructions comprising the computer program.

Other objects and features of the present invention will become apparent from the following detailed description considered in conjunction with the accompanying drawings.

In the following the solution according to the invention is described with respect to the claimed providing systems as well as with respect to the claimed methods. Features, advantages or alternative embodiments herein can be assigned to the other claimed objects and vice versa. In other words, claims for the providing systems can be improved with features described or claimed in the context of the methods. In this case, the functional features of the method are embodied by objective units of the providing system.

Furthermore, in the following the solution according to the invention is described with respect to methods and systems for processing medical data processed by an aggregate algorithm as well as with respect to methods and systems for providing an aggregate algorithm for processing medical data. Features, advantages or alternative embodiments herein can be assigned to the other claimed objects and vice versa. In other words, claims for methods and systems for respective local algorithm can be improved with features described or claimed in context of the methods and systems for processing medical data processed by an aggregate algorithm, and vice versa.

In particular, the trained function or algorithm of the methods and systems for processing medical data processed by an aggregate algorithm can be adapted by the methods and systems for providing an aggregate algorithm for processing medical data.

The drawings, however, are only exemplary and schematic solely for the purpose of illustration and do not limit the invention. The drawings show:
- Fig. 1: an exemplary embodiment of a system configured to perform exemplary embodiments of the inventive method for providing an aggregate algorithm and for processing medical data according to the present invention,
- Fig. 2: a flow chart of an exemplary embodiment of the method for providing an aggregate algorithm according to the present invention, and
- Fig. 3: the use of initial training data in an exemplary embodiment of the method for providing an aggregate algorithm according to the present invention.

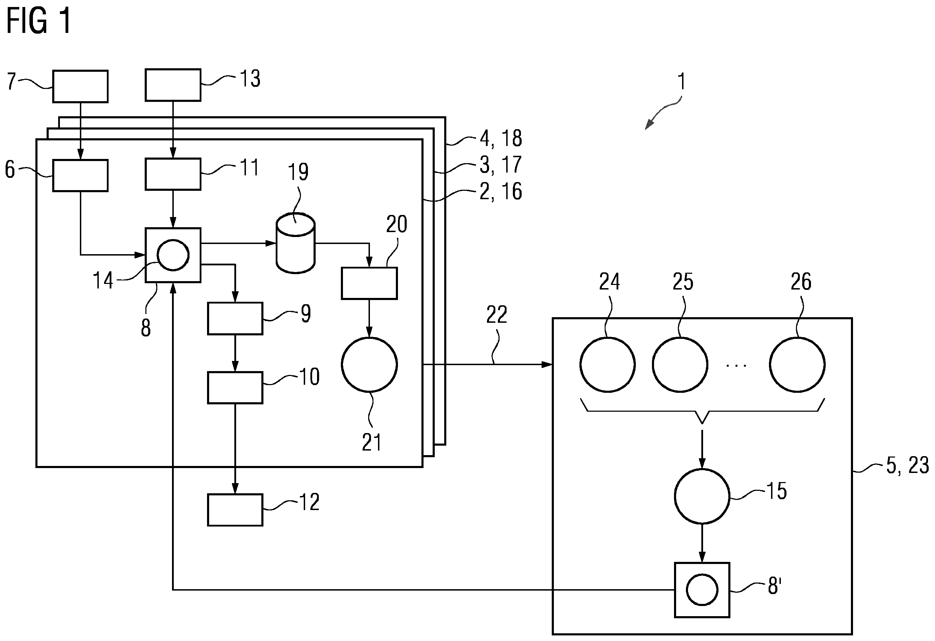

Fig. 1 shows a schematic representation of a system 1 that can be used to provide an aggregate algorithm for processing medical data and to process medical data. The system 1 comprises multiple processing units 2 - 5. The processing units 2 - 4 implement a method for processing medical data, especially medical image data. An acquisition device 7, e.g. a medical imaging device, especially a computer tomograph or a magnetic resonance tomograph, is used to provide the medical data 6 to the respective processing units 2, 3, 4. In the example the acquisition device 7 is shown as a separate unit. It is however possible to integrate the processing units 2, 3, 4 as part of the respective acquisition device 7. The medical data 6 is processed by a program 8 to a generate output data 9 and a local data representation 10 that can e.g. be output on an output device 12, especially a monitor or a similar device, to inform the user about the output data 9. The program 8 can e.g. perform a segmentation of an image that is provided as medical data 6. Additionally or alternatively certain features in the medical data 6 can be classified etc. The program can be controlled by input means 13 by a user that can especially be used to record a feedback information 11.

The generation of the output data 9 as initially performed by using an initial algorithm 14 that can e.g. be preinstalled on the processing units 2, 3, 4. As described in more detail later this initial algorithm 14 can then be replaced by an aggregate algorithm 15 that can be updated at certain times.

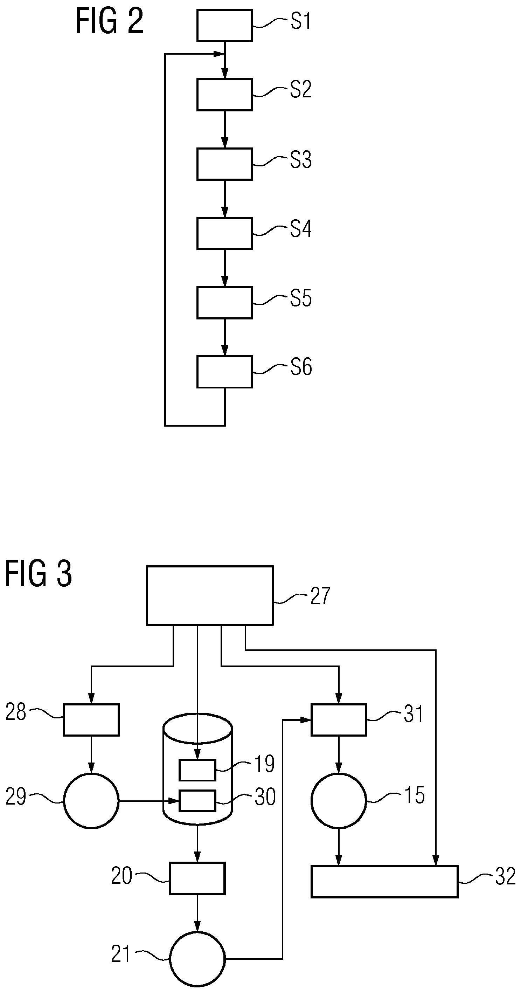

A method for providing such an aggregate algorithm 15 will now be discussed with additional reference to the flow chart shown in fig. 2.

In step S1 an initial algorithm is provided. The initial algorithm can e.g. be provided by a manufacturer a medical imaging device or another device used for medical data acquisition. The initial algorithm can be trained by machine learning. This is discussed in more detail later with reference to fig. 3.

In step S2 the initial algorithm or parameters describing the initial algorithm or a program comprising the initial algorithm is transferred to multiple local systems 16, 17, 18 that are implemented by the processing units 2, 3, 4 in the example shown in fig. 1 and that can be used to process medical data 6 as discussed above. E.g. the initial algorithm 14 can be used to perform coronary tree segmentation.

During the processing of medical data 6 local training data 19 is generated in step S3 that does not leave the local system 16, 17, 18. To generate the local training data 19 the program 8 can e.g. provide at least one user interface element that allows the user to add the current case to the local training data. The user can be allowed to provide user feedback 11 with the input means 13 e.g. to rank the performance of the currently used algorithm and/or to modify the output data 9 or the data representation 10. The rating that can e.g. range from zero to five stars, wherein five stars can represent a complete satisfaction and zero or one star a strong dislike, and/or any modifications or the result of these modifications can be stored with the respective training dataset a part of the local training data 19.

At certain times or when a certain amount of local training data 19 is acquired training by machine learning 20 can be performed in step S4 to train a local algorithm 21 in the respective local system 16, 17, 18. For example a supervised learning can be implemented to teach the local algorithm 21 to generate output data 9 that already takes into account typical modifications of the output data or the local data representation by the user. Alternatively the local algorithm can be trained by reinforcement learning, e.g. using ratings provided as a user feedback. Alternatively or additionally ratings provided by the user can be used to trainer a local algorithm 21 that is able to predict a rating for a given output data and that can later be used for reinforcement learning for the aggregate algorithm.

Preferably the local algorithm 21 is not directly used in the local system 16, 17, 18, since it is not validated yet. Instead in step S5 a respective algorithm dataset 22 describing the respective local algorithm 21 is transferred from the respective local system 16, 17, 18 to an aggregating system 23 that is implemented by the processing unit 5 in the example shown in fig. 1. The algorithm datasets 22 allow the aggregating system 23 to create local copies 24, 25, 26 of the local algorithms 21 generated in the local systems 16, 17, 18. The local algorithms 21 can therefore be used in step S6 to generate an aggregate algorithm 15 that aggregates the previous local learning. In a new version 8' of the program 8 the aggregate algorithm 15 can replace the initial algorithm 14 or a previous version of the aggregate algorithm. After a validation of the aggregate algorithm 15 and/or the version 8' of the program 8 an update can be rolled out to the local systems 16, 17, 18.

The discussed method allows the ranked and potentially manually improved processing results to be fed back into the learning process, since the feedback 11 or a user information determined from this user feedback 11 is used to generate the local training data. The local training can involve but is not restricted to reinforcement learning, which updates models by awards or punishment based on the user rating, and/or methods that take into account newly arriving knowledge, e.g. in terms of the location of manual editing operations on geometric objects like vessel centrelines or organ segmentations. This way a knowledge pool is created consisting of medical image data and accompanying improved geometric objects that can be used for training at the customer side. While this information might comprise sensitive private data the inventive method avoids the transfer of this training data 19 itself by using a local training to generate a local algorithm and only use that algorithm or an algorithm dataset describing that algorithm in the further training process outside of the local system.

The locally improved or trained local algorithms 21 can then be transferred to an aggregating system, e.g. operated by a manufacturer of medical imaging equipment, at regular intervals or once a certain amount of local training data is collected. These algorithms are then used to generate the aggregate algorithm 15 that could also be labelled as supermodel. The local algorithms and therefore also the algorithm dataset typically merely represent a machine-readable representation of the acquired knowledge and cannot be deciphered by humans or by known algorithms to recover the used local training data. It can however still be advantageous to use authentication and/or encryption for the communication between the different local systems 16, 17, 18 and the aggregating system 23.

For the generation of the aggregate algorithm 15 ensemble learning techniques can be used. It is e.g. possible to implement the aggregate algorithm 15 in such a way that all or at least some of the local algorithms are executed and the output data can then e.g. be averaged or a majority voting can be used. It is possible to only use a selection of the local algorithms 21 or to use a different weighting for the outputs of different local algorithms 21. This will be discussed in more detail later with reference to fig. 3.

The aggregate algorithm 15 can also be a higher-level algorithm consisting of a multitude of lower-level machine learning-based components. For example a coronary tracing could be implemented in such a way. The individual components can be subject to the discussed distributed approach wherein local algorithms 21 for the respective component are trained. The higher-level system can then be composed of multiple aggregate algorithms 15, each implementing a component of the more complex model.

Before the aggregate algorithm 15 is included in an updated version of a program 8' it can e.g. be validated. This will also be discussed in detail later. The updated aggregate algorithm 15 can be rolled out to be installed in the different local systems 16, 17, 18 as part of a service pack or a regular product release.

The use of aggregate algorithms that aggregate knowledge from a multitude of locally trained algorithms allows for the use of a bigger base of data than would be otherwise possible. This can improve a performance of machine learning-based systems. The resulting aggregate algorithm is especially expected to be superior to the individual local algorithms.

Fig. 3 schematically shows the use of initial training data 27 in the generation of an initial algorithm 29, the local algorithms 21 and the aggregate algorithm 15. The initial algorithm 29 can be trained by machine learning 28, using a well-known supervised learning approach that uses the initial training data 27. This is the usual approach of providing an algorithm trained by machine learning.

It is however possible to also use the initial training data 27 or at least a part 30 of an initial training data 27 in further steps during the generation of the aggregate algorithm 15.

It is for example possible to store at least a part 30 of the initial training data 27 on each of the local systems 16, 17, 18. This is especially advantageous when the local machine learning 20 is a supervised learning, since such a type of learning uses a multitude of training datasets. The storage of a part 30 of the initial training data 27 on the local systems 16, 17, 18 allows this machine learning 20 to be performed by using a large training database formed from the local training data 19 and at least a part 30 of the initial training data 27.

In the local systems 16, 17, 18 or in the aggregating system 23 a quality measure 31 that can be used as a weighting factor can be determined for each of the local algorithms 21 depending on at least part of the initial training data 27. It can especially be determined how well the individual local algorithms 21 perform when applied to input data from the initial training data 27. This information can be used to determine which of the local algorithms 21 should be used to generate the aggregate algorithm 15 and/or to determine weighting factors for the individual local algorithms 21 when they are combined to form the aggregate algorithm 15.

Once an aggregate algorithm 15 is determined a validation 32 can be performed, e.g. by applying the aggregate algorithm 15 on part of the initial training data 27. It is e.g. only possible to include the aggregate algorithm 15 in an update when its performance on the training dataset 27 is better or at least comparable to a previous version of the algorithm or the initial algorithm.

The proposed method offers an approach for the training of machine learning based classifiers or other machine learning based algorithms that are applied by end-users in the day-today routine. The users are able to contribute to the quality and performance of the used tool and will therefore directly benefit from their efforts when submitting, rating or amending data to generate the local training data. Since only data concerning the trained local algorithm is transferred to external systems, patient data does not need to leave the hospitals. Therefore there are no issues concerning data protection and no required separate anonymization step. The local algorithms and therefore the algorithm datasets do not carry any patient specific information.

Since the proposed method allows the use of user feedback to further train the algorithms there is no or at least less necessity of high quality annotations in the initial training data. The application of reinforcement learning techniques in combination with user ratings allows adding imperfect examples to the training, which will typically be based on the editing which is done by the user anyway. Therefore less in-house annotation efforts are required to improve algorithms. In-house annotations and other initial training data can e.g**.** only be used to provide an initial algorithm and for validating data versions of the algorithm.

The method allows for a larger pool of training data than would otherwise be achievable. Therefore a broader coverage of variables can be achieved. Differences in the local population can be captured in a better way during training and potentially the entire installed base could contribute to the training procedure. At the same time the combination of the local algorithms into an aggregate algorithm allow for achieving a better algorithm performance than by using only individual local models. A local validation and verification of local algorithms is not required, since this can be performed on the aggregating system.

Although the present invention has been described in detail with reference to the preferred embodiment, the present invention is not limited by the disclosed examples from which the skilled person is able to derive other variations without departing from the scope of the attached patent claims.

## Claims

1. A computer-implemented method for providing an aggregate algorithm (15) for processing medical image data (6),
- wherein said aggregate algorithm (15) generates processed medical image data output data,
- wherein a multitude of local algorithms (21) is trained by machine learning (20),
- wherein the training of each respective local algorithm (21) is performed on a respective local system (16, 17, 18) using respective local training data (19),
- wherein a respective algorithm dataset (22), which describes the complete local algorithm (21) or parameters that are used to parametrize a given basic algorithm, is transferred from a respective local system (16, 17, 18) to an aggregating system (23) that generates the aggregate algorithm (15) based on the algorithm datasets (22),
- wherein the aggregate algorithm (15) comprises the evaluation of at least some of the local algorithms (21), wherein the outputs of the individual local algorithms (21) are combined by a weighted or non-weighted averaging and/or majority voting,
wherein at least part of the local training data (19) is generated by applying an initial algorithm (14) to local input data to generate local output data (9), providing a local data representation (10) that depends on the local output data (9) to a user and storing a user feedback (11) provided by the user as part of the local training data (19), wherein medical image data (6) is processed to generate a local data representation (10) that can be output on an output device, and wherein the user feedback comprises a rating of the quality of the local output data (9) or the local data representation (10) and/or a user modification of the local output data (9) or the local data representation (10), whereby the training of each respective local algorithm (21) and therefore as a result the aggregate algorithm (15) therefore takes the user feedback (11) into account.

2. Method according to claim 1, **characterized in that** the aggregate algorithm (15) is updated in multiple iterations, wherein at least one of the iterations uses the aggregate algorithm (15) determined during a previous iteration as the initial algorithm (14) for the current iteration.

3. Method according to claim 1 or 2, **characterized in that** the initial algorithm (14) is trained by machine learning (28) using initial training data (27).

4. Method according to claim 3, **characterized in that** at least part of the initial training data (27) is provided to the aggregating system (23) and used to validate (32) the aggregate algorithm (15) and/or to determine a respective quality measure (31) for each of the local algorithms (21), wherein the quality measures (31) are used to generate the aggregate algorithm (15).

5. Method according to claim 3 or 4, **characterized in that** at least part (30) of the initial training data (27) is provided to each of the local systems (16, 17, 18) and used to train the local algorithm (21).

6. Method according to one of the preceding claims, **characterized in that** the local algorithm (21) and/or the aggregate algorithm (15) and/or the initial algorithm (14) process image data as input data and output a segmentation of the image data and/or parameters concerning the anatomy of a patient depicted by the image data.

7. Method for processing medical image data according to claim 1, comprising the step of processing medical image data, **characterized in that** the medical data (6) is processed by an aggregate algorithm (15) generated by a method according to one of the preceding claims to generate output data.

8. System comprising at least one processing unit (2 - 5) configured to perform the function of the aggregating system (23) in the method of one of the claims 1 to 6 and/or to perform the method of claim 7 to process medical image data.

9. System according to claim 8, wherein the processing unit (5) is configured to perform the function of the aggregating system (23) in the method of one of the claims 1 to 7, **characterized in that** it comprises multiple further processing units (2, 3, 4) that are each configured to perform the function of the local system (16, 17, 18) in the method of one of the claims 1 to 6 and/or to perform the method of claim 7 to process medical image data.

10. Computer program that can be directly loaded into a memory unit of a processing unit (2 - 5), the computer program comprising instructions for performing the function of the aggregating system (23) in the method of one of the claims 1 to 6 and/or for performing the method of claim 7 to process medical image data when the program is executed on the processing unit (2 - 5).

11. Computer-readable storage medium containing electronically readable instructions comprising the computer program according to claim 10.

## Patentansprüche

1. Computerimplementiertes Verfahren zur Bereitstellung eines Aggregatalgorithmus (15) zur Verarbeitung medizinischer Bilddaten (6),
- wobei der Aggregatalgorithmus (15) verarbeitete medizinische Bilddaten-Ausgabedaten erzeugt,
- wobei eine Mehrzahl von lokalen Algorithmen (21) durch maschinelles Lernen (20) trainiert wird,
- wobei das Training jedes jeweiligen lokalen Algorithmus (21) auf einem jeweiligen lokalen System (16, 17, 18) unter Verwendung jeweiliger lokaler Trainingsdaten (19) durchgeführt wird,
- wobei ein jeweiliger Algorithmusdatensatz (22), der den vollständigen lokalen Algorithmus (21) oder Parameter beschreibt, die zum Parametrisieren eines gegebenen Basisalgorithmus verwendet werden, von einem jeweiligen lokalen System (16, 17, 18) zu einem Aggregationssystem (23) übertragen wird, das den Aggregatalgorithmus (15) basierend auf den Algorithmusdatensätzen (22) erzeugt,
- wobei der Aggregatalgorithmus (15) die Bewertung von mindestens einigen der lokalen Algorithmen (21) umfasst, wobei die Ausgaben der einzelnen lokalen Algorithmen (21) durch eine gewichtete oder nicht gewichtete Mittelwertbildung und/oder Mehrheitsabstimmung kombiniert werden,
wobei mindestens ein Teil der lokalen Trainingsdaten (19) dadurch erzeugt wird, dass ein Anfangsalgorithmus (14) auf lokale Eingabedaten angewendet wird, um lokale Ausgabedaten (9) zu erzeugen, einem Benutzer eine lokale Datendarstellung (10), die von den lokalen Ausgabedaten (9) abhängt, bereitgestellt wird und eine durch den Benutzer bereitgestellte Benutzerrückmeldung (11) als Teil der lokalen Trainingsdaten (19) gespeichert wird, wobei medizinische Bilddaten (6) verarbeitet werden, um eine lokale Datendarstellung (10) zu erzeugen, die auf einer Ausgabevorrichtung ausgegeben werden kann, und wobei die Benutzerrückmeldung eine Bewertung der Qualität der lokalen Ausgabedaten (9) oder der lokalen Datendarstellung (10) und/oder eine Benutzermodifikation der lokalen Ausgabedaten (9) oder der lokalen Datendarstellung (10) umfasst, wodurch das Training jedes jeweiligen lokalen Algorithmus (21) und somit der Aggregatalgorithmus (15) die Benutzerrückmeldung (11) berücksichtigen.

2. Verfahren nach Anspruch 1, **dadurch gekennzeichnet, dass** der Aggregatalgorithmus (15) in mehreren Iterationen aktualisiert wird, wobei mindestens eine der Iterationen den während einer vorherigen Iteration bestimmten Aggregatalgorithmus (15) als Anfangsalgorithmus (14) für die aktuelle Iteration verwendet.

3. Verfahren nach Anspruch 1 oder 2, **dadurch gekennzeichnet, dass** der Anfangsalgorithmus (14) durch maschinelles Lernen (28) unter Verwendung von anfänglichen Trainingsdaten (27) trainiert wird.

4. Verfahren nach Anspruch 3, **dadurch gekennzeichnet, dass** zumindest ein Teil der anfänglichen Trainingsdaten (27) dem Aggregationssystem (23) bereitgestellt und verwendet wird, um den Aggregatalgorithmus (15) zu validieren (32) und/oder ein jeweiliges Qualitätsmaß (31) für jeden der lokalen Algorithmen (21) zu bestimmen, wobei die Qualitätsmaße (31) verwendet werden, um den Aggregatalgorithmus (15) zu erzeugen.

5. Verfahren nach Anspruch 3 oder 4, **dadurch gekennzeichnet, dass** zumindest ein Teil (30) der anfänglichen Trainingsdaten (27) jedem der lokalen Systeme (16, 17, 18) bereitgestellt und zum Trainieren des lokalen Algorithmus (21) verwendet wird.

6. Verfahren nach einem der vorhergehenden Ansprüche, **dadurch gekennzeichnet, dass** der lokale Algorithmus (21) und/oder der Aggregatalgorithmus (15) und/oder der Anfangsalgorithmus (14) Bilddaten als Eingangsdaten verarbeiten und eine Segmentierung der Bilddaten und/oder Parameter bezüglich der Anatomie eines durch die Bilddaten dargestellten Patienten ausgeben.

7. Verfahren zur Verarbeitung medizinischer Bilddaten nach Anspruch 1, umfassend den Schritt der Verarbeitung medizinischer Bilddaten, **dadurch gekennzeichnet, dass** die medizinischen Daten (6) durch einen Aggregatalgorithmus (15) verarbeitet werden, der durch ein Verfahren nach einem der vorhergehenden Ansprüche erzeugt wird, um Ausgabedaten zu erzeugen.

8. System, das mindestens eine Verarbeitungseinheit (2 - 5) umfasst, die dazu ausgelegt ist, die Funktion des Aggregationssystems (23) in dem Verfahren nach einem der Ansprüche 1 bis 6 durchzuführen und/oder das Verfahren nach Anspruch 7 durchzuführen, um medizinische Bilddaten zu verarbeiten.

9. System nach Anspruch 8, wobei die Verarbeitungseinheit (5) dazu ausgelegt ist, die Funktion des Aggregationssystems (23) in dem Verfahren nach einem der Ansprüche 1 bis 7 durchzuführen, **dadurch gekennzeichnet, dass** sie mehrere weitere Verarbeitungseinheiten (2, 3, 4) umfasst, die jeweils dazu ausgelegt sind, die Funktion des lokalen Systems (16, 17, 18) in dem Verfahren nach einem der Ansprüche 1 bis 6 durchzuführen und/oder das Verfahren nach Anspruch 7 durchzuführen, um medizinische Bilddaten zu verarbeiten.

10. Computerprogramm, das direkt in eine Speichereinheit einer Verarbeitungseinheit (2 - 5) geladen werden kann, wobei das Computerprogramm Anweisungen zum Durchführen der Funktion des Aggregationssystems (23) in dem Verfahren nach einem der Ansprüche 1 bis 6 und/oder zum Durchführen des Verfahrens nach Anspruch 7 zum Verarbeiten medizinischer Bilddaten umfasst, wenn das Programm auf der Verarbeitungseinheit (2 - 5) ausgeführt wird.

11. Computerlesbares Speichermedium, das elektronisch lesbare Anweisungen enthält, die das Computerprogramm nach Anspruch 10 umfassen.

## Revendications

1. Un procédé mis en œuvre par ordinateur pour donner un algorithme (15) d'agrégat pour le traitement d'une donnée (6) d'image médicale,
- dans lequel ledit algorithme (15) d'agrégat crée une donnée de sortie traitée de données d'image médicale,
- dans lequel une multitude d'algorithmes (21) locaux subit un apprentissage par apprentissage (20) automatique,
- dans lequel l'apprentissage de chaque algorithme (21) local respectif est effectué sur un système (16, 17, 18) local respectif utilisant une donnée (19) d'apprentissage locale respective,
- dans lequel un ensemble (22) de données d'algorithme respectif, qui décrit l'algorithme (21) local complet ou des paramètres, qui sont utilisés pour paramétrer un algorithme de base donné, est transféré d'un système (16, 17, 18) local respectif à un système (23) d'agrégation, qui crée l'algorithme (15) d'agrégat sur la base des ensembles (22) de données d'algorithme,
- dans lequel l'algorithme (15) d'agrégat comprend l'évaluation d'au moins certains des algorithmes (21) locaux, dans lequel les sorties des algorithmes (21) locaux individuels sont combinées en faisant une moyenne pondérée ou non pondérée et/ou par un vote à la majorité,
dans lequel au moins une partie de la donnée (19) d'apprentissage locale est créée en appliquant un algorithme (14) initial à une donnée d'entrée locale pour créer une donnée (9) de sortie locale, donnant une représentation (10) de donnée locale, qui dépend de la donnée (9) de sortie locale à un utilisateur en mettant en mémoire une réaction (11) de l'utilisateur donnée par l'utilisateur comme partie de la donnée (19) d'apprentissage locale, dans lequel une donnée (6) d'image médicale est traitée pour créer une représentation (10) de donnée locale, qui peut être sortie sur un dispositif de sortie, et dans lequel la réaction de l'utilisateur comprend une évaluation de la qualité de la donnée (9) de sortie locale ou de la représentation (10) de la donnée locale et/ou une modification par l'utilisateur de la donnée (9) de sortie locale ou de la représentation (10) de donnée locale, dans lequel l'apprentissage de chaque algorithme (21) local respectif et donc en résultat l'algorithme (15) d'agrégat prend ainsi en compte la réaction (11) de l'utilisateur.

2. Procédé suivant la revendication 1, **caractérisé en ce que** l'algorithme (15) d'agrégat est mis à jour en de multiples itérations, dans lequel au moins l'une des itérations utilise l'algorithme (15) d'agrégat déterminé pendant une itération précédente comme algorithme (14) initial pour l'itération en cours.

3. Procédé suivant la revendication 1 ou 2, **caractérisé en ce que** l'algorithme (14) initial subit un apprentissage par apprentissage (28) automatique en utilisant la donnée (27) d'apprentissage initiale.

4. Procédé suivant la revendication 3, **caractérisé en ce qu'**au moins une partie de la donnée (27) d'apprentissage initiale est donnée au système (23) d'agrégation et utilisée pour valider (32) l'algorithme (15) d'agrégat et/ou pour déterminer une mesure (31) de qualité respective pour chacun des algorithmes (21) locaux, dans lequel les mesures (31) de qualité sont utilisées pour créer l'algorithme (15) d'agrégat.

5. Procédé suivant la revendication 3 ou 4, **caractérisé en ce qu'**au moins une partie (30) de la donnée (27) d'apprentissage initiale est donnée à chacun des systèmes (16, 17, 18) locaux et utilisée pour faire l'apprentissage de l'algorithme (21) local.

6. Procédé suivant l'une des revendications précédentes, **caractérisé en ce que** l'algorithme (21) local et/ou l'algorithme (15) d'agrégat et/ou l'algorithme (14) initial traite la donnée d'image comme une donnée d'entrée et sort une segmentation de la donnée d'image et/ou des paramètres concernant l'anatomie d'un patient dépeinte par la donnée d'image.

7. Procédé de traitement d'une donnée d'image médicale suivant la revendication 1, comprenant le stade de traitement d'une donnée d'image médicale, **caractérisé en ce que** la donnée (6) médicale est traitée par un algorithme (15) d'agrégat créé par un procédé suivant l'une des revendications précédentes pour créer une donnée de sortie.

8. Système comprenant au moins une unité (2 à 5) de traitement configurée pour effectuer la fonction du système (23) d'agrégation dans le procédé de l'une des revendications 1 à 6 et/ou pour effectuer le procédé de la revendication 7 pour traiter une donnée d'image médicale.

9. Système suivant la revendication 8, dans lequel l'unité (5) de traitement est configurée pour effectuer la fonction du système (23) d'agrégation dans le procédé de l'une des revendications 1 à 7, **caractérisé en ce qu'**il comprend de multiples autres unités (2, 3, 4) de traitement, chacune configurée pour effectuer la fonction du système (16, 17, 18) local dans le procédé de l'une des revendications 1 à 6 et/ou pour effectuer le procédé de la revendication 7 pour traiter une donnée d'image médicale.

10. Programme d'ordinateur, qui peut être chargé directement dans une unité de mémoire d'une unité (2 à 5) de traitement, le programme d'ordinateur comprenant des instructions pour effectuer la fonction du système (23) d'agrégation dans le procédé de l'une des revendications 1 à 6 et/ou pour effectuer le procédé de la revendication 7 pour traiter une donnée d'image médicale, lorsque le programme est exécuté sur l'unité (2 à 5) de traitement.

11. Support de mémoire déchiffrable par ordinateur contenant des instructions déchiffrables électroniquement comprenant le programme d'ordinateur suivant la revendication 10.
